(19) Europäisches Patentamt
European Patent Office
Office européen des brevets

(11) EP 0 727 218 A2

# (12) EUROPEAN PATENT APPLICATION

(43) Date of publication:
21.08.1996 Bulletin 1996/34

(51) Int. Cl.[6]: **A61K 31/70**, A61K 35/78

(21) Application number: 96101937.9

(22) Date of filing: 09.02.1996

(84) Designated Contracting States:
AT BE CH DE DK ES FR GB GR IE IT LI LU MC NL PT SE

(30) Priority: 10.02.1995 JP 45050/95
01.09.1995 JP 247025/95

(71) Applicant: SUNTORY LIMITED
Kita-ku, Osaka-shi, Osaka (JP)

(72) Inventors:
- Nakahara, Koichi
Toyonaka-shi, Osaka (JP)
- Miyagawa, Katsuro
Takatsuki-shi, Osaka (JP)
- Kodama, Tohru
Takatsuki-shi, Osaka (JP)
- Fujii, Wataru
Ibaraki-shi, Osaka (JP)

(74) Representative: Kolb, Helga, Dr. Dipl.-Chem. et al
Hoffmann, Eitle & Partner,
Patent-und Rechtsanwälte,
Arabellastrasse 4
81925 München (DE)

## (54) Anti-allergic composition containing god-type ellagitannin as active ingredient

(57) Anti-allergic composition containing a GOD-type ellagitannin as an active ingredient, as well as foods and pharmaceuticals incorporating said anti-allergic composition. The composition has the ability to inhibit histamine release from mast cells and hence is useful as an anti-inflammatory or anti-allergic composition. By regular taking of foods that contain the anti-allergic composition, allergic diseases such as allergic rhinitis, atopic dermatitis and asthma can be effectively prevented or ameliorated.

EP 0 727 218 A2

## Description

This invention relates to anti-allergic compositions, more particularly, to anti-allergic compositions containing a GOD-type ellagitannin as an active ingredient, as well as foods and pharmaceuticals containing said anti-allergic compositions.

The term "anti-allergic activity" to be used herein refers to the ability to ameliorate, alleviate, inhibit or prevent allergic symptoms.

Many studies have been conducted on pharmaceutical ingredients having an anti-inflammatory or anti-allergic activity and numerous synthetic compounds have been reported to possess such activities. However, natural substances generally recognized as safe, for example, food, food ingredient having the anti-inflammatory or anti-allergic activity are limited to licorice (in particular, glycyrrhetic acid) and other ingredients and no safe natural substances that have satisfactory anti-inflammatory and anti-allergic activities have yet been obtained.

If safe natural substances having anti-inflammatory and anti-allergic activities were obtained, they could be administered with increased safety to many patients complaining of allergic symptoms. In addition, if they were regularly eaten as foods rather than administered as pharmaceuticals, the valuable safe natural substances could be taken into the body not only in an easy way but also as a preventive measure against the manifestation of the symptoms. In spite of these demands, there have not been known any safe natural substances that have anti-inflammatory and anti-allergic activities and, hence, no useful food materials have yet been provided.

Thus, a need has existed for providing materials that can be incorporated in foods for regular intake and use and which are capable of ameliorating or preventing inflammations or symptoms associated with allergies.

Allergy are generally classified into four types, anaphylaxis (type I), cytotoxic (type II), Arthus (type III) and delayed or cellular-mediated (type IV). Pollinosis which is a great concern in Japan today is classified as a type I immediate allergy. Atopic dermatitis is said to involve a type I reaction as the principal mechanism but recent studies have shown that a type IV allergy is also a factor.

The type I allergy is hypothesized to occur by the following mechanism: the IgE antibody produced in response to an antigen binds to an Fc receptor on the mast cell membrane; when another antigen crosslinks with the IgE antibody, histamine and other chemical substances in granules in the mast cell are released, thereby causing, either directly or indirectly, acute inflammatory reactions that are accompanied by asthma or rhinitis. Therefore, the type I allergy could be prevented by blocking either one of the stages of this pathway. Diseases that are manifested by the type I allergy include anaphylactic shock, most allergic bronchial asthma, urticaria, allergic rhinitis (with complications such as hypersecretion of pituita and nasal congestion) and intestinal allergy.

Sensitized lymphocytes participate in the type IV (delayed) allergy and among them, delayed hypersensitive effector T cells react with antigens to produce and release various soluble active factors, thereby inducting inflammatory reactions such as the transmigration of macrophages and neutrophils and enhanced permeability of blood vessels. The type IV allergy is "delayed type" since it takes 24 - 48 h for the inflammatory reactions to reach a peak. Symptoms that are manifested by the type IV allergy include the tuberculin reaction, contact dermatitis, allograft rejection, tuberculosis, thyroadenitis and allergic encephalitis.

The present inventors noted histamine release inhibitors as substances that inhibit the type I allergic reactions and the resulting acute inflammatory reactions, and conducted an extensive search to locate a safe natural substance having an excellent histamine release inhibiting activity. As a result, it was found that GOD-type ellagitannins contained in Rosaceae have a histamine release inhibiting action.

In order to test the GOD-type ellagitannins for their effect on the type IV allergy, the present inventors caused contact dermatitis in the pinna of the ear of a mouse and measured the degree of edema that developed in the pinna, as well as the permeability of pinnal blood vessels. The inventors also conducted a mouse delayed podedema test on the GOD-type ellagitannins. As a result, the GOD-type ellagitannins exhibited an anti-allergic activity comparable to those of existing anti-allergic drugs, demonstrating their effectiveness against the type IV allergies.

Thus, the present inventors found that GOD-type ellagitannins had a sufficient anti-allergic activity to be useful as anti-allergic compositions. The present invention has been accomplished on the basis of this finding.

An object, therefore, of the invention is to provide an anti-allergic composition containing a GOD-type ellagitannin as an active ingredient.

Another object of the invention is to provide a food product containing said anti-allergic composition.

A further object of the invention is to provide a pharmaceutical composition containing said anti-allergic composition.

Fig. 1 shows a sequence for the production of a GOD-type ellagitannin from the extract of *Rubus suavissimus* S. Lee with hot water in Preparation Example 1;

Fig. 2 shows the results of HPLC analysis on the hydrolyzate of the GOD-type ellagitannin produced in Preparation Example 1;

Fig. 3 shows a sequence for the production of a GOD-type ellagitannin from the extract of *Rubus suavissimus* S. Lee with hot water in Preparation Example 3;

Fig. 4 is a graph showing the histamine release inhibiting activities of TCE, TCF3, TCF5 and TCF11 obtained in Preparation Example 3;

Fig. 5 is a graph showing the histamine release inhibiting activities of an ellagitannin monomer, a GOD-type ellagitannin dimer and a GOD-type ellagitannin polymer;

Fig. 6 is a graph showing the result of the test conducted in Example 4 to evaluate the effect of GOD-type ellagitannin on allergic rhinitis;

Fig. 7 is a graph showing the effectiveness of GOD-type ellagitannin in inhibiting the weight gain of the pinna of the ear of mice in which contact dermatitis was caused in Example 5;

Fig. 8 is a graph showing the effectiveness of GOD-type ellagitannin in inhibiting the hyperpermeation of pinnal blood vessels of mice in which contact dermatitis was caused in Example 5; and

Fig. 9 is a graph showing the effectiveness of GOD-type ellagitannin in inhibiting the mouse delayed podedema induced by injection of sheep red blood cells in Example 6.

The GOD-type ellagitannin which is an active ingredient of the anti-allergic composition of the invention (said GOD-type ellagitannin is hereunder referred to as "GOD ellagitannin" for short) is a compound having a structural unit represented by the following formula (I):

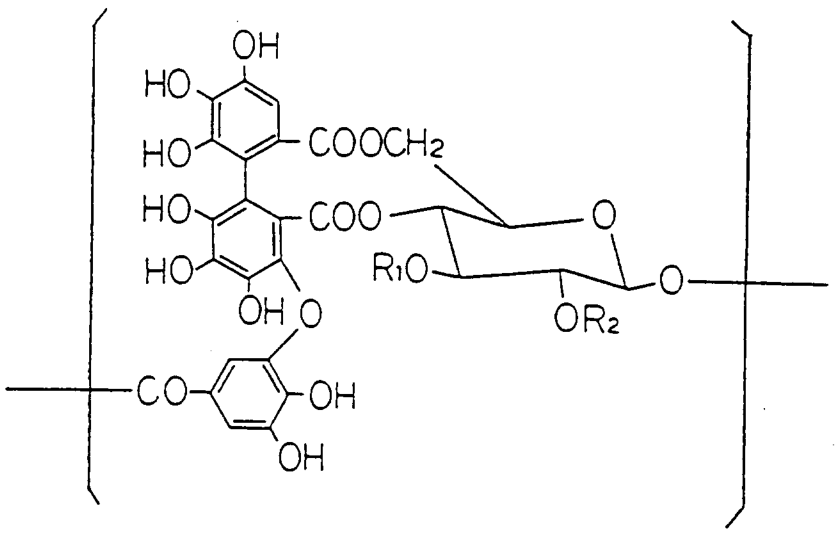

(I)

(where $R_1$ and $R_2$ each represents a hydrogen atom or a galloyl group or, when taken together, they represent a hexahydroxydiphenoyl group).

In addition to the saccharide structure, the structural unit contains a partial structure formed by ether bonding between the hexahydroxydiphenoyl group (hereunder sometimes abbreviated to D) and the galloyl group (hereunder sometimes abbreviated to G) as shown below:

(galloyl group)

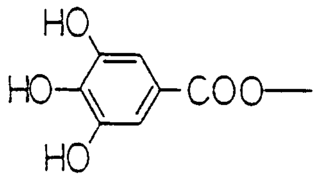

(hexahydroxydiphenoyl group)

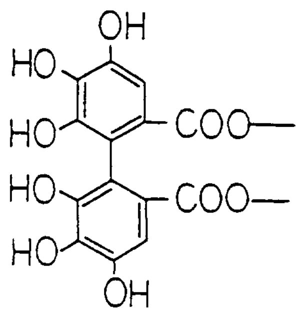

(partial structure)

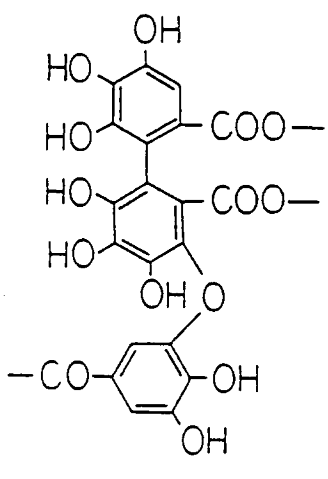

The oxygen in the ether bond in this partial structure is supplied from the G side and hence said partial structure is called a GOD type (G → O → D type, with O designating an oxygen atom).

The molecular weight of the GOD ellagitannin of the invention and the degree of its polymerization are not limited to any particular values as long as it has the above-defined structural unit. The molecular weights of Ellagitannin monomer and dimer from Tien-cha (*Rubus suavissims* S.Lee.) extract wherein GOD ellagitannin contained were determined by FAB-MS using a Jeol JMS-HX110/110A tandem mass spectrometry system.

For estimation of molecular weight of polyphenol polymer, these compounds were methylated by diazomethane. The apparent molecular weight of the methylated active compounds from Tien-cha extract were determined by comparing the elution profiles on a TSK gel G3000H-XL (Tosoh Co., Tokyo Japan) column (7.5 × 300 mm) in comparison to those of polystyrens of known molecular weight. Preferably, it has a molecular weight of no more than 20,000 with the degree of polymerization not more than 20. A particularly preferred GOD ellagitannin is represented by the following

formula (II):

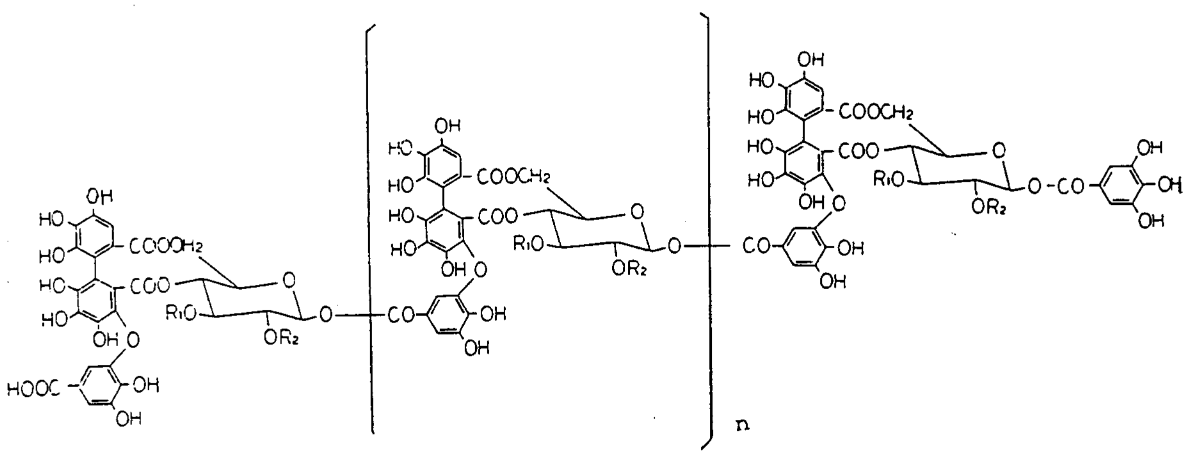

(II)

(where $R_1$ and $R_2$ have the same meanings as defined above; n is a number of from 0 to 20).

Another preferred GOD ellagitannin is represented by the following formula (III):

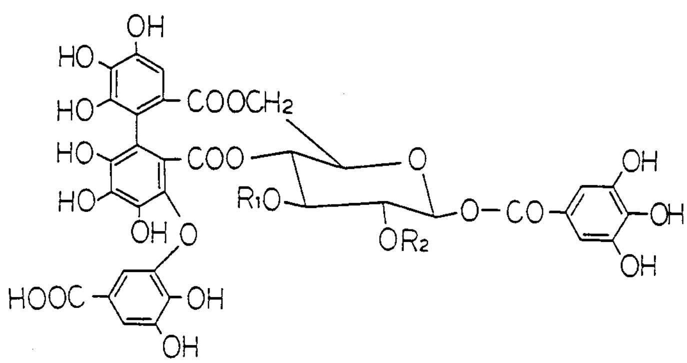

(III)

(where $R_1$ and $R_2$ have the same meanings as defined above).

The GOD ellagitannins mentioned above are known compounds and include the following: the GOD ellagitannin obtained from *di-yu* which is a crude drug prepared by drying the rhizome of *Sanguisorba officinalis* L. as described in Nonaka et al., J. C. S. Perkin I, 1067-1073 (1982); the GOD ellagitannin obtained from *Rosa henryi* BOUL. as described in Yoshida et al., Chem. Pharm. Bull. 40(8), 1997-2001 (1992); and the GOD ellagitannin obtained from Rubus and Sanguisorba such as *R.crataegifolius* BUNGE, *R.parvifolius* L., *R.palmatus* THUNB, *R.sieboldii* BLUME, *R.corcborifolius* L., *R.palmatus* THUNB. var *coptophyllus* KUNZE, *R.idaeus* L., *R.mesogeanus* FOCKE, *R.phoenicolasius* MAXIM., *R.calycinoides* HAYATO, *R.lambertianus* SERINGE, *S.tenuifolia var parviflora* MAXIM. and *Rubus chingii* as described in Tanaka et al., Chem. Pharm. Bull. 41(7), 1214-1220 (1193). These GOD ellagitannins are useful as an active ingredient in the anti-allergic composition of the invention.

The GOD ellagitannins that can be used in the invention are by no means limited to those described in the references listed above and also useful are those GOD ellagitannins that were extracted and purified from other plants that contain the compound of interest.

The plants from which the GOD ellagitannin can be extracted are not limited to any particular types and, besides the plants used as starting materials in the references listed above, other plants of the rose family may be employed, among which the following are particularly mentioned: *Rubus microphyllus*, *R.koehncanus*, *R.trifidus*, *R.medius*, *R.acuminatus*, *R.ellipticus*, *R.multibureatus*, *R. coreanus*, *R.foliosus*, *R.parvifolius*, *Rubus suavissimus* S. Lee, *Rubus babae* Naruhashi, *Rubus fuiticosus* L., *Rubus hiraseanus* Makino, *Rubus hirsutus* Thunb., *Rubus masakii* Naruhashi, *Rubus medius* O.Ktze., *Rubus nigakuma* Oka et Naruhashi, *Rubus nikaii* Ohwi, *Rubus ohmineanus* Koidz., *Rubus*

pedatus Smith, *Rubus pseudochingii* Naruhashi, *Rubus tawadanus* Koidz., *Rubus toyorensis* Koidz., *Rubus trifidus* Thunb. ex Murrey, *Rubus utchinensis* Koidz., *Rubus yenoshimanu* Koidz., and *Sanguisorba tenuifolia* Fisch. var. *alba* Trautv. et Meyer.

To extract GOD ellagitannins from the plants mentioned above, various solvents may be used and they include water, alcohols such as methanol and ethanol, and ketones such as acetone. These solvents may be used individually or in admixtures. Since the extracts are eventually incorporated in foods and other products, the use of water, alcohols and mixtures thereof is preferred from a safety viewpoint.

The ratio between the plant and the extracting solvent is in no way limited but the solvent is preferably used in amounts 2 - 1,000 times as much as the plant, with 5 - 100 times being particularly preferred from the viewpoints of ease of extraction and its efficiency. The extraction temperature is conveniently selected from the range of room temperature to the boiling point of the solvent at atmospheric pressure. The extraction time varies with the extraction temperature and other factors but is preferably selected from several seconds to two days.

The plant extracts thus prepared may be worked up to GOD ellagitannins of higher purity by subjecting them to various purification techniques such as treatment with adsorbents, membrane separation and solvent fractionation.

To purify the active ingredient of the plant extracts, the plant extracts may be treated with any solvents that are not highly miscible with the GOD ellagitannin and which are separable from water; preferred solvents include butanol, ethyl acetate and methyl acetate. Another workup procedure that can be adopted is fractionation by molecular weight using an ultrafiltration (UF) membrane.

Alternatively, the plant extract or products thereof by fractionation with solvents may be subjected to adsorption column chromatography and eluted with one or more solvents to provide GOD ellagitannins of higher purity.

Adsorptive partitioning by adsorption column chromatography may proceed as follows: the plant extract or products of its fractionation with solvents are dissolved in a small amount of a solvent such as water, methanol or ethanol or a mixture thereof; the solution is adsorbed on an adsorbent in column such as Sephadex LH-20 (Pharmacia, Sweden), Diaion HP20 (Mitsubishi Kasei Corp.), Develosil ODS (Nomura Chemical Co.), Sepabeads HP1MG (Mitsubishi Kasei Corp.) or Toyopearl HW40F (Tosoh Corp.), washed thoroughly with water, and eluted with a hydrophilic solvent such as methanol, ethanol or acetone or a mixture thereof. GOD ellagitannins of much higher purity can be obtained by tandem adsorption column chromatography.

Furthermore, the GOD ellagitannins that can be used in the invention, are obtained from the shredded plants containing the GOD ellagitannins. The shredded plants may be used as an ingredient, or may be prepared a form suitable for extraction such as tea bag. The shredded leaves, stems or roots are preferably used as shredded plants.

The thus prepared GOD ellagitannins may be formulated as the anti-allergic composition of the invention either as such or by combining them with known pharmaceutical vehicles.

The anti-allergic composition of the invention may be formulated as oral drugs such as tablets, granules, powders and syrups, or as parenteral drugs such as injections and suppositories, or even as food additives. Any pharmaceutical vehicles that are commonly used in accordance with the above-mentioned dosage forms may be employed without any particular limitations.

While many various pharmaceutical vehicles may be used, preferred examples include: solid vehicles such as starch, lactose, saccharose, mannitol, carboxymethylcellulose, corn starch and inorganic salts; liquid vehicles such as distilled water, physiological saline, glucose in aqueous solution, alcohols (e.g. ethanol) propylene glycol and polyethylene glycol; various animal and vegetable oils; and oil vehicles such as white petrolatum, paraffin and waxes.

If the anti-allergic composition of the invention is to be used as pharmaceuticals such as anti-inflammatory and anti-allergic drugs that rely mainly upon the histamine release inhibiting action for its efficacy the dose of administration of the GOD ellagitannin varies greatly with its purity, the age of the patient, the severity of the disease and other factors. For peroral administration, the dose varies typically from 0.5 to 5,000 mg per day on the basis of the GOD ellagitannin in a pure form.

The food product of the invention can be included with the anti-allergic composition of the invention which is used as a food additive. The food product included the anti-allergic composition of the invention may also incorporate generally food ingredients including glucose, fructose, sucrose, maltose, sorbitol, stevioside, rubusoside, corn syrup, lactose, citric acid, tartaric acid, malic acid, succinic acid, lactic acid, L-ascorbic acid, dl-$\alpha$-tocopherol, sodium erythorbite, glycerin, propylene glycol, glycerin fatty acid esters, polyglycerin fatty acid esters, sucrose fatty acid esters, sorbitan fatty acid esters, propylene glycol fatty acid esters, gum arabic, carrageenan, casein, gelatin, pectin, agar, vitamin Bs, nicotinic acid amide, calcium pantothenate, amino acids, calcium salts, dyes, flavoring agents and preservativies. Examples of the food products that can be produced in this way include candies, chewing gum, processed milk, yogurt, whey drinks, drinking yogurt, juice, various other beverages, ice creams, puddings and sweetened agar jelly (mizu yokan).

The GOD ellagitannin which is the active ingredient of the anti-allergic composition of the invention is also contained in Tien-cha (dried the leaves or stems of *Rubus suavissimus* S. Lee) which has been taken habitually as tea in the southern part of the People's Republic of China and, hence, it has been generally recognized as safe at all. When it is to be incorporated in foods, its concentration should desirably be adjusted to range from 0.0001 to 5.0% on the basis of the GOD ellagitannin in a pure form.

The GOD ellagitannin which is the active ingredient of the anti-allergic composition of the invention inhibits the release of histamine from mast cells in the type I allergic reactions, thereby inhibiting the allergic reactions and the resulting allergic inflammations. In this way, the GOD ellagitannin exhibits therapeutic or preventive effects on the diseases caused by allergic inflammations such as allergic rhinitis and atopic dermatitis due to cedar pollens, house dust and other allergens, as well as allergic diseases such as allergic asthma and food allergy.

The inventors previously found that the extract of Tien-cha (*Rubus suavissimus* S. Lee) had efficient anti-inflammatory and anti-allergic activities (see Unexamined Published Japanese Patent Application (kokai) No. 192114/1994) and it is speculated that the active ingredient of the extract is the GOD ellagitannin of the invention.

The anti-allergic composition of the invention is capable of inhibiting the release of histamine from mast cells. The composition also gave satisfactory results in tests on the type IV allergy. Therefore, the composition is useful as a pharmaceutical composition for ameliorating or inhibiting allergic symptoms.

The anti-allergic composition of the invention has the added advantage that if foods containing said composition as a food additive are regularly taken in, it becomes possible to amelionate or prevent the diseases caused by allergic inflammations such as allergic rhinitis and atopic dermatitis due to cedar pollens, house dust and other allergens, as well as allergic diseases such as allergic asthma and food allergy.

The following preparation examples for the GOD ellagitannin and the associated working examples are provided for the purpose of further illustrating the present invention but are in no way to be taken as limiting.

<u>Preparation Example 1</u>

Preparating GOD ellagitannin from Tien-cha (1):

A hundred grams of Tien-cha leaves were put into a 3,000-ml conical flask. Following the addition of hot water (1,0000 ml), the tea leaves were subjected to extraction in a boiling water bath. The extract was filtered and the filtrate was freeze-dried to yield 32 g of a Tien-cha extract (which is hereunder referred to as TCE).

A portion (23 g) of the Tie-cha extract was dissolved in 2 L of water and subjected to solvent fractionation with the same amounts of ethyl acetate and n-butanol in that order to yield 15 g of a water-soluble fraction (which is hereunder referred to as TCF-3). The fraction was dissolved in 500 ml of water, adsorbed on a column (4 cm∅ × 32 cm) of Diaion HP20 (Mitsubishi Kasei Corp.), washed with 2 L of water and eluted with 2 L of 60 vol% ethanol in water to yield 5.6 g of 60% ethanol eluted fraction (which is hereunder referred to as TCF5). The fraction was dissolved in 100 ml of water-ethanol (1:1 v/v), adsorbed on a column (4 cm∅ × 30 cm) of Sephadex LH-20 (Pharmacia, Sweden), washed with 1 L of water, eluted with 1 L each of 50 vol% ethanol in water and ethanol in that order and further eluted with 70 vol% acetone in water to yield 1.7 g of a 70% acetone eluted fraction (which is hereunder referred to as TCF11). These procedures of preparation and purification are shown schematically in Fig. 1.

A portion (5 mg) of TCF11 (70% acetone eluted fraction) was dissolved in 2 ml of 1N HCl, heated at 100°C for 2 h and subjected to three extractions with 2 ml of ethyl acetate. The extract was analyzed by HPLC and the results are shown in Fig. 2. Three peaks appeared at retention times of 5.275 min (sanguisorbic acid dilactone, GOD), 2.283 min (gallic acid, G) and 8.492 min (ellagic acid, D). Thus, the formation of GOD-type ellagitannin was verified. TCF11 contained the GOD ellagitannin at a concentration of 90%.

The conditions for HPLC analysis were as follows: the column was packed with Develosil ODS-5 (4.5 mm∅ × 150 mm; product of Nomura Chemical Co.), which was eluted with a solvent system consisting of 0.05M monopotassium phosphate, 0.05M phosphoric acid, ethanol and ethyl acetate (40:40:15:5 v/v), with uv detection conducted at 280 nm.

<u>Preparation Example 2</u>

Preparing GOD ellagitannin from Tien-cha (2):

Ten grams of Tien-cha extract with hot water was dissolved in 1 L of water and subjected to solvent fractionation with the same amounts of ethyl acetate and n-butanol in that order to yield 6.58 g of a water-soluble fraction. A portion (1 g) of the fraction was dissolved in 5 ml of water, adsorbed on a column (4 cm∅ × 30 cm) of Develosil ODS-10 (Nomura Chemical Co.), washed with 2 L of water, and eluted with 2 L of 20 vol% ethanol in water to yield 0.36 g of a 20% ethanol eluted fraction.

The formation of a GOD ellagitannin in the 20% ethanol eluted fraction from Develosil ODS-10 was verified by the same method as in Preparation Example 1.

Preparation Example 3

Preparing GOD ellagitannin from Tien-cha (3):

Tien-cha leaves were subjected to extraction with hot water as in Preparation Example 1. A portion (23 g) of the extract was fractionated as in Preparation Example 1 by the procedures illustrated in Fig. 3 to yield four fractions, TCE, TCF3, TCF5 and TCF11.

Example 1

Assay for the Histamine Release Inhibiting Activity (1):

The GOD ellagitannins produced in Preparation Examples 1 and 2 were assayed for their histamine release inhibiting activity by the following method. The results are shown in Table 1.

(Inhibition of histamine release from rat peritoneal mast cells)

Male Wistar rats (Nippon Charles River) weighing 272 - 349 g were acclimated for 5 days or longer and used as test animals. The rats were kept in a cage controlled at a temperature of 20 - 25°C and a humidity of 40 - 70% with daily illumination for 12 h (the light turned on at 7 a.m. and turned off at 7 p.m.) The animals were fed *ad libitum* a solid diet MF (Oriental Yeast Co., Ltd.) and tap water that had been sterilized by exposure to uv light after filtration through a filter of 5 µm.

Mast cells were collected from the peritoneal cavities of the rats as follows in accordance with the method of Sullivan et al. [J. Immunol., 114, 1473-1479 (1975)]. Male Wistar rats (8 wk old) were beheaded and bled to death. Twenty milliliters of a $Ca^{2+}$ free mast cell medium (MCM solution*) were injected into the peritoneal cavity of each animal and the abdominal part of each animal was lightly massaged before the liquid peritoneal infusion was recovered. Mast cells were isolated from the infusion by multi-layered centrifugation using BSA-physiological saline (specific gravity, 1.068). The isolated mast cells were washed twice with a MCM solution and suspended in a MCM solution to give a concentration of $2 \times 10^4$ cells per milliliter.

A histamine release inhibition test was conducted in the following manner. A portion (0.5 ml) of the cell suspension ($2 \times 10$ cells/ml) was added to 5 µl of a sample solution and the mixture was preincubated at 37°C for 10 min. Then, 5 µl of compound 48/80 (50 µg/ml) was added as a degranulation inducer and the mixture was incubated at 37°C for 10 min to initiate reaction. Thereafter, the reaction mixture was cooled with ice to stop the reaction. After centrifugation at 3000 x g for 2.5 min at 4°C, the supernatant was subjected to high-performance liquid chromatography using a fluoroscence detector to determine the level of histamine released from the cells into the supernatant.

The histamine release inhibiting activity was calculated from the measured histamine level by the following equation:

$$\text{Percent histamine release supperssion} = \frac{1\text{-(SR-C)}}{\text{(R-C)}} \times 100$$

where

C : the level of histamine released from control cells in the absence of inducer
R : the level of histamine released from cells in the presence of inducer
SR: the level of histamine released from cells in the presence of both inducer and test sample.

* MCM solution: 6.7 mM phosphate buffer solution (pH 7.1) containing 140 mM NaCl, 2.5 mM KCl, 1.2 mM $MgCl_2$, 5 mM glucose and 0.1% bovine serum albumin (BSA)

Table 1

| Test Sample | Histamine release inhibiting activity, $IC_{85}$ (ppm) |
|---|---|
| Sodium cromoglycate | 300** |
| Tien-cha extract with hot water (Preparation Example 1) | 140 |
| 60% Ethanol eluted fraction from Diaion HP-20(Preparation Example 1) | 12.5 |
| 70% Acetone eluted fraction from Sephadex LH-20 (Preparation Example 1) | 7.5 |
| Tien-cha extract with hot water (Preparation Example 2) | 72.6 |
| Water-soluble fraction (Preparation Example 2) | 18.5 |
| 20% Ethanol eluted fraction from Develosil ODS-10 (Preparation Example 2) | 10.0 |

* $IC_{85}$: concentration for 85% inhibition of histamine release
** Sodium cromoglycate achieved only 50% inhibition of histamine release at 300 ppm and 85% inhibition was impossible to accomplish at higher doses.

Example 2

Assay for the Histamine Release Inhibiting Activity (2):

As in Example 1, fractions TCE, TCF3, TCF5 and TCF11 which were obtained in Preparation Example 3 were evaluated for their respective histamine release inhibiting activities. The results are shown in Fig. 4.

Example 3

Assay for the Histamine Release Inhibiting Activity (3):

The extract of Tien-cha (*Rubus Suavissimus* S. Lee) with hot water was treated by HPLC to recover an ellagitannin monomer (isostrictinin; Mw = 634) and a GOD ellagitannin dimer (Mw = 1886). TCF11 was similarly treated by HPLC to recover a GOD ellagitannin polymer having a molecular weight of at least 3000. The conditions of HPLC were as follows:

| | |
|---|---|
| column: | Develosil ODS-5 (4.6 × 150 mm) |
| Solvent: | 0.05M $H_3PO_4$:0.05M $KH_2$ $PO_4$:EtOH:AcOEt = 40:40:15:5 |
| Flow Rate: | 1.0 ml/min |
| Detection: | UV 280 nm |

The three samples, ellagitannin monomer, GOD ellagitannin dimer and GOD ellagitannin polymer, were evaluated for their ability to inhibit the histamine release from rat peritoneal mast cells as inducted by compound 48/80. The results are shown in Fig. 5.

As one can see from Fig. 5, each sample inhibited the release of histamine in a dose-dependent manner and the inhibiting activity increased markedly with the increasing degree of polymerization of ellagitannin. Stated specifically, the monomer had an $IC_{50}$ of 50 µM, the dimer 4 µM and the polymer 0.6 µM, indicating that the dimer and the polymer were 12 and 83 times as active as the monomer. Compared with a known anti-allergic drug, ketotifen, the dimer and the polymer were 100 and 667 times more effective.

Evaluation was also conducted on epigallocatechin gallate and catechin which were two principal ingredients of polyphenols in green tea. The inhibiting activity of epigallocatechin gallate was weak ($IC_{50}$ = 150 µM) and catechin was totally ineffective.

Example 4

Effect of GOD Ellagitannin on Allergic Rhinitis:

1. Preparing passively sensitized rat model

Anti-DNP-As serum (serum IgE titer = 1:32) were injected in respective amounts of 0.1 ml into the skin of both buccal parts of unsensitized rats and under the nasorstal skin. The thus sensitized rats were subjected to the following experiment 48 h later.

2. Inducing allergy in the nose

The passively sensitized rats were fasted overnight. Under anesthetization with urethane (administered intraperitoneally at a dose of 12.5 mg/kg), each animal was incised in the neck along the midline and a cannula was inserted into the trachea for sustained respiration and the esophagus was ligated. One end of a polyethylene tube was inserted into the nasal cavity through the incision. The other end of the polyethylene tube was connected to a perfusion pump and heated (37°C) physiological saline was perfused at a flow rate of 0.25 ml/min to wash the interior of the nasal cavity. Following intravenous injection of 1% Evans blue in an amount of 0.5 ml/100 g of body weight, the perfusion was sampled for 10 min (period 1). Subsequently, an aqueous solution of DNP-As in physiological saline (2 mg/ml) was perfused for 10 min (period 2) to remove the antigen from within the nasal cavity. Thereafter, physiological saline was perfused for 30 min as fraction were sampled at 10-min intervals (periods 3, 4 and 5). In order to prevent the leakage of the perfusion, the buccal cavity of each animal was filled with a glycerin-impregnated absorbent cotton. The collected samples were centrifuged at 3,000 rpm for 10 min and the absorbance at 620 nm was measured with a spectrophotometer to evaluate the permeability of blood vessels in the nasal cavity.

In the test, GOD ellagitannin (TCF11) was administered perorally for four consecutive days starting on the day before sensitization. The control group was given only an equal volume of water. The positive control group was administered a single dose of tranilast in an amount of 300 mg/kg one hour before the induction of the reaction. The results are shown in Fig. 6, from which one can see that the efficacy of TCF11 was almost comparable to that of tranilast in the presence of an antigen (period 2); upon removal of the antigen, TCF11 proved to be more effective than tranilast in ameliorating the symptoms (periods 3, 4 and 5). It was thus clear that the GOD ellagitannin was effective against allergic rhinitis.

Example 5

Effect of GOD Ellagitannin on Mouse Pinnal Contact Dermatitis:

ICR mice were injected on the back subcutaneously with 100 µl of 2.5% dinitrofluorobenzene (DNFB of Seikagaku Kogyo Co., Ltd., Tokyo) in ethanol. Six days after this sensitization, 25 µl of 1% DNFB in oil olive was applied to both sides of the pinna of each animal so as to induce primary pinnal contact dermatitis. Twenty-four hours later, the amount of pinnal edema and the permeability of pinnal blood vessels were determined by the following methods. For determining pinnal edema, the central portion of the pinna was punched out by means of a leather punch (8 mm∅) and its weight was measured. To determine the amount of edema, the weight of a control pinna which was not coated with the antigen may be subtracted from the measured value. For determining the permeability of blood vessels, a 1% aqueous solution of Evans blue in physiological saline (0.1 ml/10 g) was administered to a tail vein and the color change that occurred in the pinna due to the spreading of Evans blue was evaluated by the following method of Katayama et al. in Katayama, S. Shinoyama, H. Ohtake, S: A New Method for Extraction of Extravasated Dye in the Skin and Influence of Fasting Stress on Passive Cutaneous Anaphylaxis in Guinea Pigs and Rats, Microbiol. Immunol. 22:89-101, 1978: the pinna was immersed in 0.5 ml of 1N KOH in aqueous solution at 37°C, left to stand for 24 h, stirred after addition of 4.5 ml of an extract (with 5:13 mixture of 0.6N $H_3PO_4$ and acetone) and centrifuged at 2700 x g for 15 min at room temperature. The resulting supernatant was measured for absorbance at 620 nm and the amount of dye leakage was calculated. The amount of the dye Evans blue was expressed in µg/ear.

The GOD ellagitannin (TCF11) was administered perorally for seven consecutive days starting the day before the subcutaneous sensitization with DNFB until pinnal contact dermatitis was induced. The control group was only administered an equal volume of pure water and the positive control group was administered perorally a single dose of Dexamethasone (Sigma) immediately before the reaction was induced.

The results are shown in Figs. 7 and 8, from which one can see that TCF11 was almost comparable in efficacy to the dexamethasone administered to the positive control group. It was thus clear that the GOD ellagitannin was effective against contact dermatitis.

Example 6

Mouse Delayed Podedema Test with GOD Ellagitannin:

ICR mice were sensitized by intravenous injection with sheep red blood cells (SRBC) at a concentration of $10^7$ cells/0.2 ml. Four days later, $10^8$ cells of SRBC were administered under the skin of the left posterior limb to induce a delayed podedema reaction. Twenty-four hours after the induction, the degree of edema was measured. GOD ellagitannin (TCF11) was administered perorally for four consecutive days starting on the day of sensitization with SRBC until podedema was induced. The control group was only administered an equal volume of pure water. The positive control group was perorally administered a single dose of Dexamethasone (Sigma) in an amount of 1 mg/kg.

The result is shown in Fig. 9, from which one can see that TCF11 was almost comparable in efficacy to dexamethasone administered to the positive control group. It was therefore clear that the GOD ellagitannin was effective against type IV allergies represented by the tuberculin reaction.

Example 7

Tablet:

Tablets were prepared using a GOD ellagitannin which was the 60% ethanol eluted fraction from Diaion HP20 (see Preparation Example 1). Stated more specifically, 150 g of that 60% ethanol eluted fraction from Diaion HP20 was mixed with an equal amount of lactose and 5 g of magnesium stearate. The mixture was tableted with a single-action tableting machine to prepare tablets each having a diameter of 10 mm and weighing 300 mg.

Patients suffering from pollinosis took two tablets daily for a week and symptoms such as itching of the eyes and hypersecretion of pituita were substantially alleviated.

Example 8

Granule:

Tablets were prepared as in Example 7, then ground, passed through a coarse screen and sieved through a fine screen to yield granules ranging from 20 - 50 mesh in size.

Example 9

Candy;

Candies were prepared from the formulation set forth below. Despite the incorporation of GOD ellagitannin, the candies had good taste without any unpleasant bitterness.

| Formula | Content (%) |
|---|---|
| Granulated sugar | 55.0 |
| Starch syrup | 43.5 |
| Citric acid | 1.0 |
| Flavoring agent | 0.2 |
| Coloring agent | 0.2 |
| GOD ellagitannin* | 0.1 |

* GOD ellagitannin: 70% acetone eluted fraction from Sephadex LH-20 in Preparation Example 1

Example 10

Juice:

Juice was prepared from the formulation set forth below. It contained a GOD ellagitannin but neither its taste nor color was adversely affected by the GOD ellagitannin.

| (Formula) | Content (%) |
|---|---|
| Juice of tangerine UNSHU in the form of a frozen concentrate | 5.0 |
| Liquid mixture of fructose and glucose | 11.0 |
| Citric acid | 0.2 |
| L-ascorbic acid | 0.02 |
| Flavoring agent | 0.2 |
| Coloring agent | 0.1 |
| GOD ellagitannin* | 0.2 |
| Water | 83.28 |

* GOD ellagitannin: 70% acetone eluted fraction from Sephadex LH-20 in Preparation Example 1

## Claims

1. An anti-allergic composition containing a GOD-type ellagitannin as an active ingredient.

2. An anti-allergic composition according to claim 1, wherein the GOD-type ellagitannin has a structural unit represented by the following formula (I):

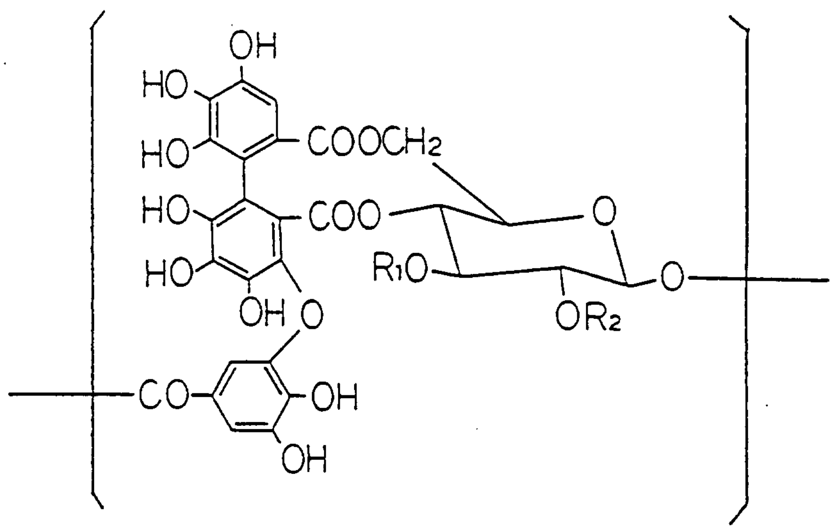

(I)

(where $R_1$ and $R_2$ each represents a hydrogen atom or a galloyl group or, when taken together, they represent a hexahydroxydiphenoyl group).

3. An anti-allergic composition according to claim 1, wherein the GOD-type ellagitannin is a compound represented by the following formula (II):

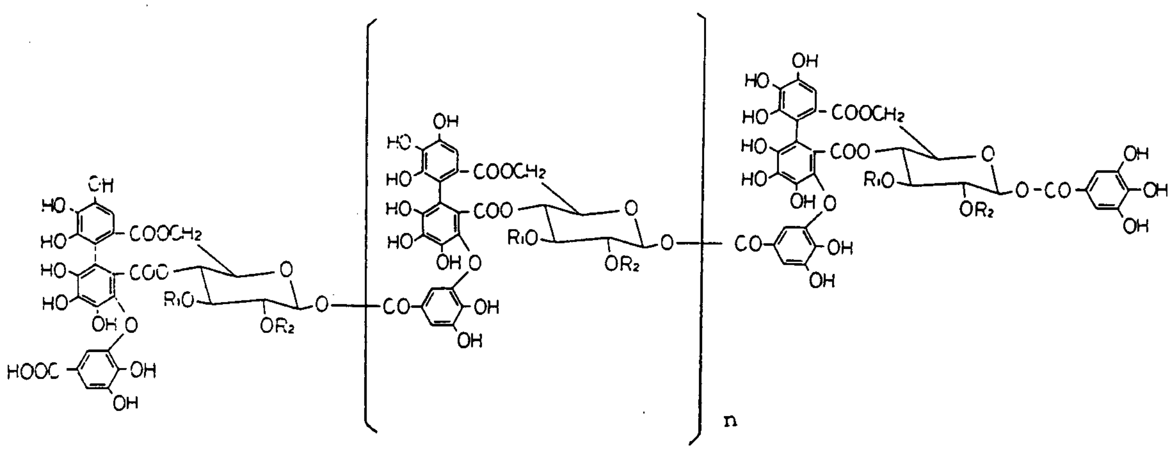

(II)

(where $R_1$ and $R_2$ have the same meanings as defined above; n is a number of from 0 to 20).

**4.** An anti-allergic composition according to claim 1, wherein the GOD-type ellagitannin is a compound represented by the following formula (III):

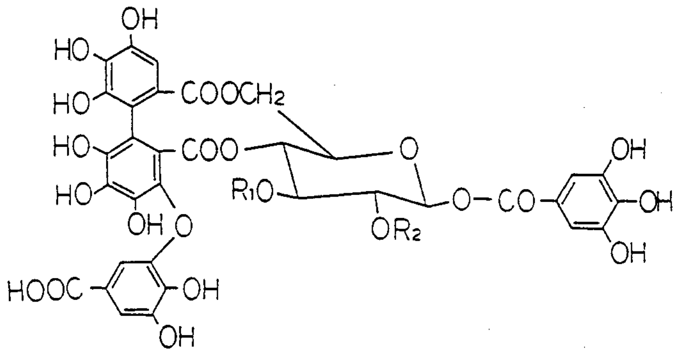

(III)

(where $R_1$ and $R_2$ have the same meanings as defined above).

**5.** An anti-allergic composition according to claim 1, wherein the GOD-type ellagitannin is an extract from a Rosaceae.

**6.** An anti-allergic composition according to claim 1, wherein the GOD-type ellagitannin is shredded Rosaceae.

**7.** An anti-allergic composition according to claim 6, wherein the shredded Rosaceae is shredded leaves, stems or roots.

**8.** An anti-allergic composition according to claim 6, wherein the shredded Rosaceae is prepared a form suitable for extraction.

**9.** An anti-allergic composition according to claim 5 or 6, wherein the Rosaceae is selected from the group of genera consisting of Rubus, Sanguisorba and Rosa.

**10.** An anti-allergic composition according to claim 5 or 6, wherein the *Rosaceae* is selected from the group consisting of *Rubus suavissimus* S. Lee, *Sanguisorba officinalis* L., *Rubus chingii*, *Rubus coreanus*, *Rosa henryi* BOUL., *Rubus crataegifolius* BUNGE, *Rubus parvifolius* L., *Rubus palmatus* THUNB., *Rubus sieboldii* BLUME, *Rubus corchorifolius* L., *Rubus palmatus* THUNB. var *coptophyllus* KUNZE, *Rubus idaeus* L., *Rubus mesogeanus*

FOCKE, *Rubus phoenicolasius* MAXIM., *Rubus calycinoides* HAYATO, *Rubus lambertianus* SERINGE, *Sanguisorba tenuifolia* var *parviflora* MAXIM., *Rubus babae* Naruhashi, *Rubus fruticosus* L., *Rubus hiraseanus* Makino, *Rubus hirsutus* Thunb., *Rubus masakii* Naruhashi, *Rubus medius* O. Ktze., *Rubus nigakuma* Oka et Naruhashi, *Rubus nikaii* Ohwi, *Rubus ohmineanus* Koidz., *Rubus pedatus* Smith, *Rubus pseudochingii* Naruhashi, *Rubus tawadanus* Koidz., *Rubus toyorensis* Koidz., *Rubus trifidus* Thunb. ex Murrey, *Rubus utchinensis* Koidz., *Rubus yenoshimanus* Koidz., and *Sanguisorba tenuifolia* Fisch var. *alb*a Trautv. et Meyer.

**11.** A composition for ameliorating or inhibiting type I allergic symptoms which contains the anti-allergic composition of any of claims 1 - 10.

**12.** A composition for ameliorating or inhibiting type IV allergic symptoms which contains the anti-allergic composition of any of claims 1- 10.

**13.** A histamine release inhibitor containing the anti-allergic composition of any of claims 1 - 10.

**14.** A food product containing the anti-allergic composition of any of claims 1 - 10.

**15.** A composition for ameliorating or inhibiting symptoms of allergic rhinitis which contains the anti-allergic composition of any of claims 1 - 10.

**16.** A composition for ameliorating or inhibiting symptoms of pollinosis which contains the anti-allergic composition of any of claims 1 - 10.

**17.** A composition for ameliorating or inhibiting symptoms of allergic symptoms due to house dust, fungi, mites, the hair of pet animals, their skin, feces and the like which contains the anti-allergic composition of any of claims 1 - 10.

**18.** A composition for ameliorating or inhibiting symptoms related to the hypersecretion of pituita, which contains the anti-allergic composition of any of claims 1 - 10.

**19.** A composition for ameliorating or inhibiting symptoms related to contact dermatitis, which contains the anti-allergic composition of any of claims 1 - 10.

**20.** A composition for ameliorating or inhibiting symptoms related atopic dermatitis, which contains the anti-allergic composition of any of claims 1 - 10.

**21.** A composition for ameliorating or inhibiting symptoms related to allergic asthma, which contains the anti-allergic composition of any of claims 1 - 10.

**22.** A composition for ameliorating or inhibiting symptoms related to food allergy, which contains the anti-allergic composition of any of claims 1 - 10.

**23.** A composition for ameliorating or inhibiting symptoms related to intestinal allergy, which contains the anti-allergic composition of any of claims 1 - 10.

**24.** A composition for ameliorating or inhibiting symptoms related to urticaria which contains the anti-allergic composition of any of claims 1 - 10.

**25.** The use of an anti-allergic composition according to any of claims 1 to 10 for the preparation of an anti-allergic composition useful against type I allergic symptoms.

**26.** The use of an anti-allergic composition according to any of claims 1 to 10 for the preparation of an anti-allergic composition useful against type IV allergic symptoms.

**27.** The use of an anti-allergic composition according to any of claims 1 to 10 for the preparation of an anti-allergic composition useful against releasing a histamine.

**28.** The use of an anti-allergic composition according to any of claims 1 to 10 for the preparation of an anti-allergic composition useful against allergic rhinitis.

29. The use of an anti-allergic composition according to any of claims 1 to 10 for the preparation of an anti-allergic composition useful against pollinosis.

30. The use of an anti-allergic composition according to any of claims 1 to 10 for the preparation of an anti-allergic composition useful against allergic symptoms due to house dust, fungi, mites, the hair of pet animals, their skin, feces and the like.

31. The use of an anti-allergic composition according to any of claims 1 to 10 for the preparation of an anti-allergic composition useful against symptoms related to the hypertension of pituita.

32. The use of an anti-allergic composition according to any of claims 1 to 10 for the preparation of an anti-allergic composition useful against symptoms related to contact dermatitis.

33. The use of an anti-allergic composition according to any of claims 1 to 10 for the preparation of an anti-allergic composition useful against symptoms related to atopic dermatitis.

34. The use of an anti-allergic composition according to any of claims 1 to 10 for the preparation of an anti-allergic composition useful against symptoms related to allergic asthma.

35. The use of an anti-allergic composition according to any of claims 1 to 10 for the preparation of an anti-allergic composition useful against symptoms related to food allergy.

36. The use of an anti-allergic composition according to any of claims 1 to 10 for the preparation of an anti-allergic composition useful against symptoms related to intestinal allergy.

37. The use of an anti-allergic composition according to any of claims 1 to 10 for the preparation of an anti-allergic composition useful against symptoms related to urticaria.

38. The use of an anti-allergic composition according to any of claims 1 to 10 in a food product which is effective against type I allergic symptoms.

39. The use of an anti-allergic composition according to any of claim 1 to 10 in a food product which is effective against type IV allergic symptoms.

40. The use of an anti-allergic composition according to any of claims 1 to 10 in a food product which is effective against releasing a histamine.

41. The use of an anti-allergic composition according to any of claim 1 to 10 in a food product which is effective against allergic rhinitis.

42. The use of an anti-allergic composition according to any of claims 1 to 10 in a food product which is effective against pollinosis.

43. The use of an anti-allergic composition according to any of claims 1 to 10 in a food product which is effective against allergic symptoms due to house dust, fungi, mites, the hair of pet animals, their skin, feces and the like.

44. The use of an anti-allergic composition according to any of claims 1 to 10 in a food product which is effective against symptoms related to the hypersecretion of pituita.

45. The use of an anti-allergic composition according to any of claim 1 to 10 in a food product which is effective against symptoms related to contact dermatitis.

46. The use of an anti-allergic composition according to any of claim 1 to 10 in a food product which is effective against symptoms related to atopic dermatitis.

47. The use of an anti-allergic composition according to any of claims 1 to 10 in a food product which is effective against symptoms related to allergic asthma.

**48.** The use of an anti-allergic composition according to any of claim 1 to 10 in a food product which is effective against symptoms related to food allergy.

**49.** The use of an anti-allergic composition according to any of claims 1 to 10 in a food product which is effective against symptoms related to intestinal allergy.

**50.** The use of an anti-allergic composition according to any of claims 1 to 10 in a food product which is effective against symptoms related to urticaria.

Fig. 1

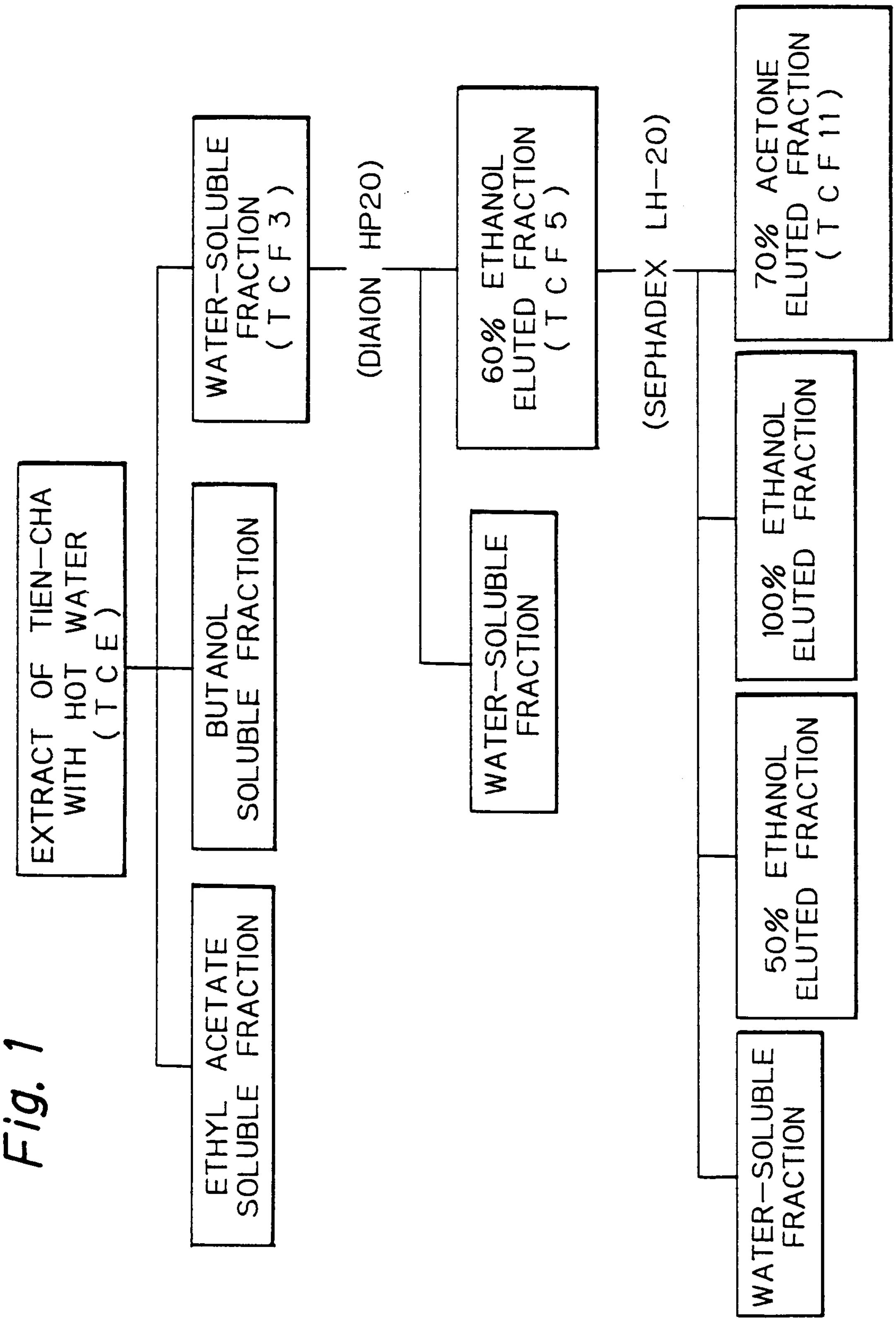
EXTRACT OF TIEN-CHA WITH HOT WATER (T C E)
ETHYL ACETATE SOLUBLE FRACTION
BUTANOL SOLUBLE FRACTION
WATER-SOLUBLE FRACTION (T C F 3)
(DIAION HP20)
WATER-SOLUBLE FRACTION
60% ETHANOL ELUTED FRACTION (T C F 5)
(SEPHADEX LH-20)
WATER-SOLUBLE FRACTION
50% ETHANOL ELUTED FRACTION
100% ETHANOL ELUTED FRACTION
70% ACETONE ELUTED FRACTION (T C F 11)

*Fig. 2*

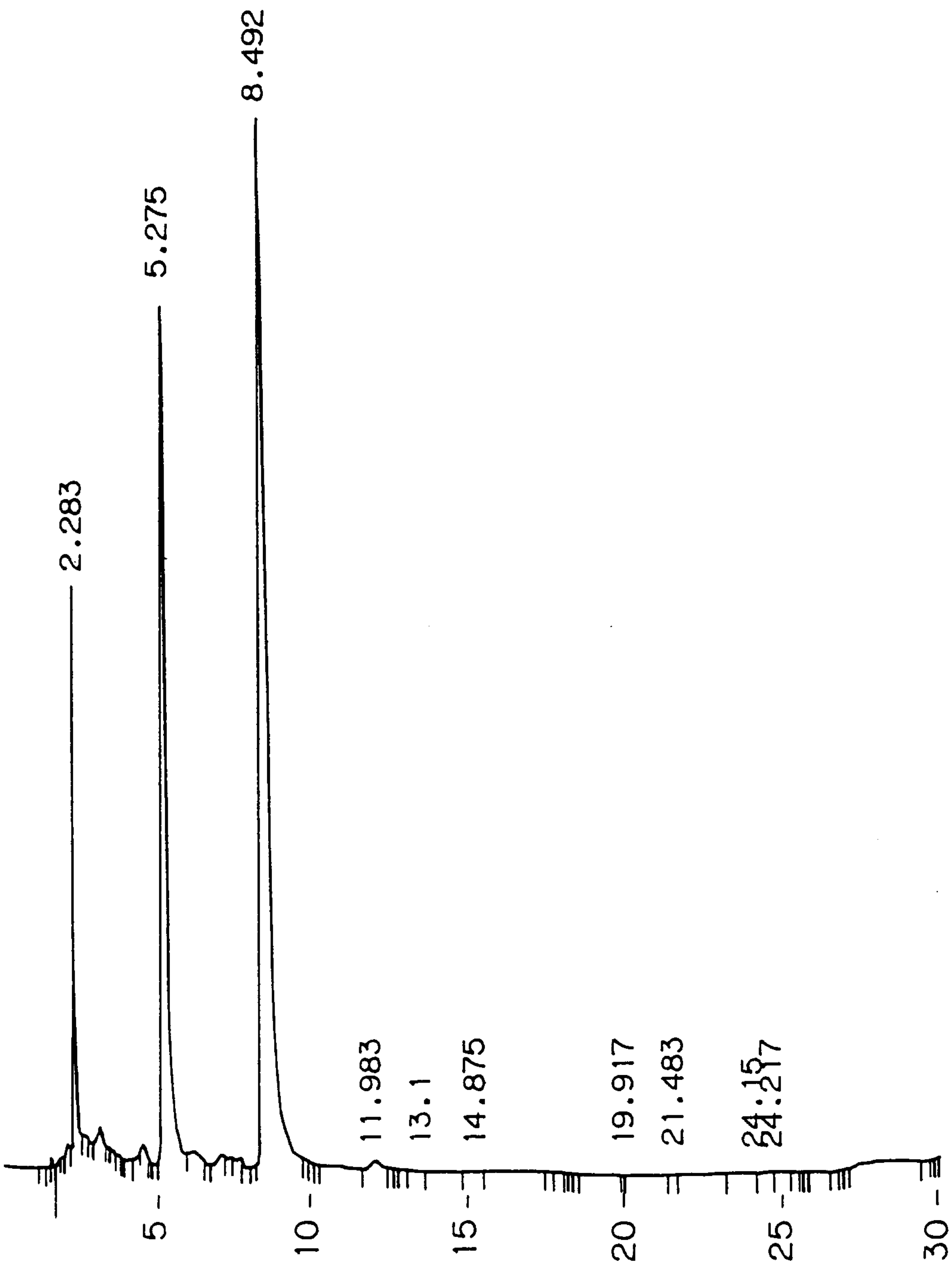
2.283
5.275
8.492
11.983
13.1
14.875
19.917
21.483
24.15
24.217
5
10
15
20
25
30

Fig. 3

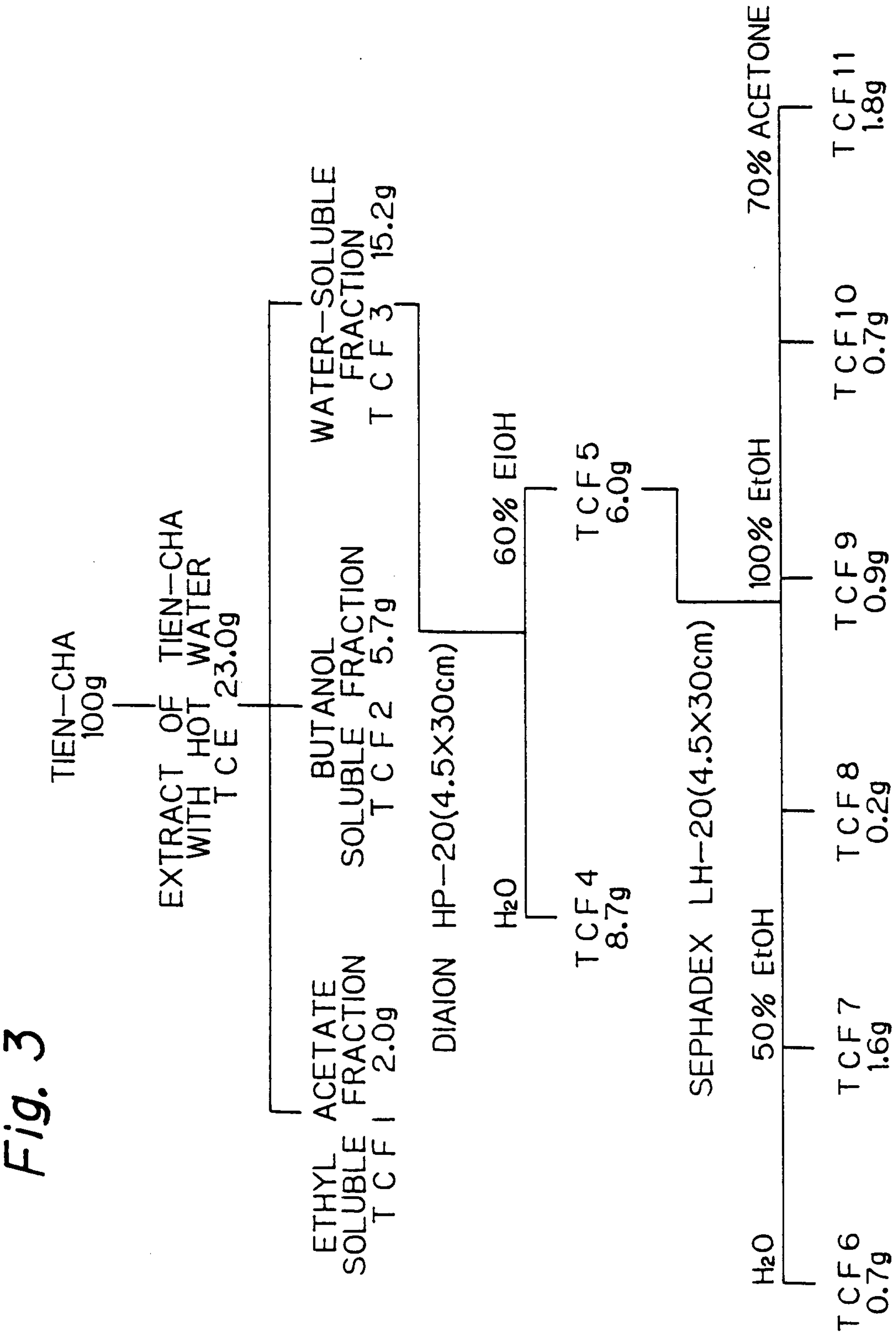
TIEN-CHA
100g
EXTRACT OF TIEN-CHA
WITH HOT WATER
T C E 23.0g
ETHYL ACETATE
SOLUBLE FRACTION
T C F 1 2.0g
BUTANOL
SOLUBLE FRACTION
T C F 2 5.7g
WATER-SOLUBLE
FRACTION
T C F 3 15.2g
DIAION HP-20(4.5X30cm)
H2O
60% EtOH
T C F 4
8.7g
T C F 5
6.0g
SEPHADEX LH-20(4.5X30cm)
H2O
50% EtOH
100% EtOH
70% ACETONE
T C F 6
0.7g
T C F 7
1.6g
T C F 8
0.2g
T C F 9
0.9g
T C F 10
0.7g
T C F 11
1.8g

*Fig. 4*

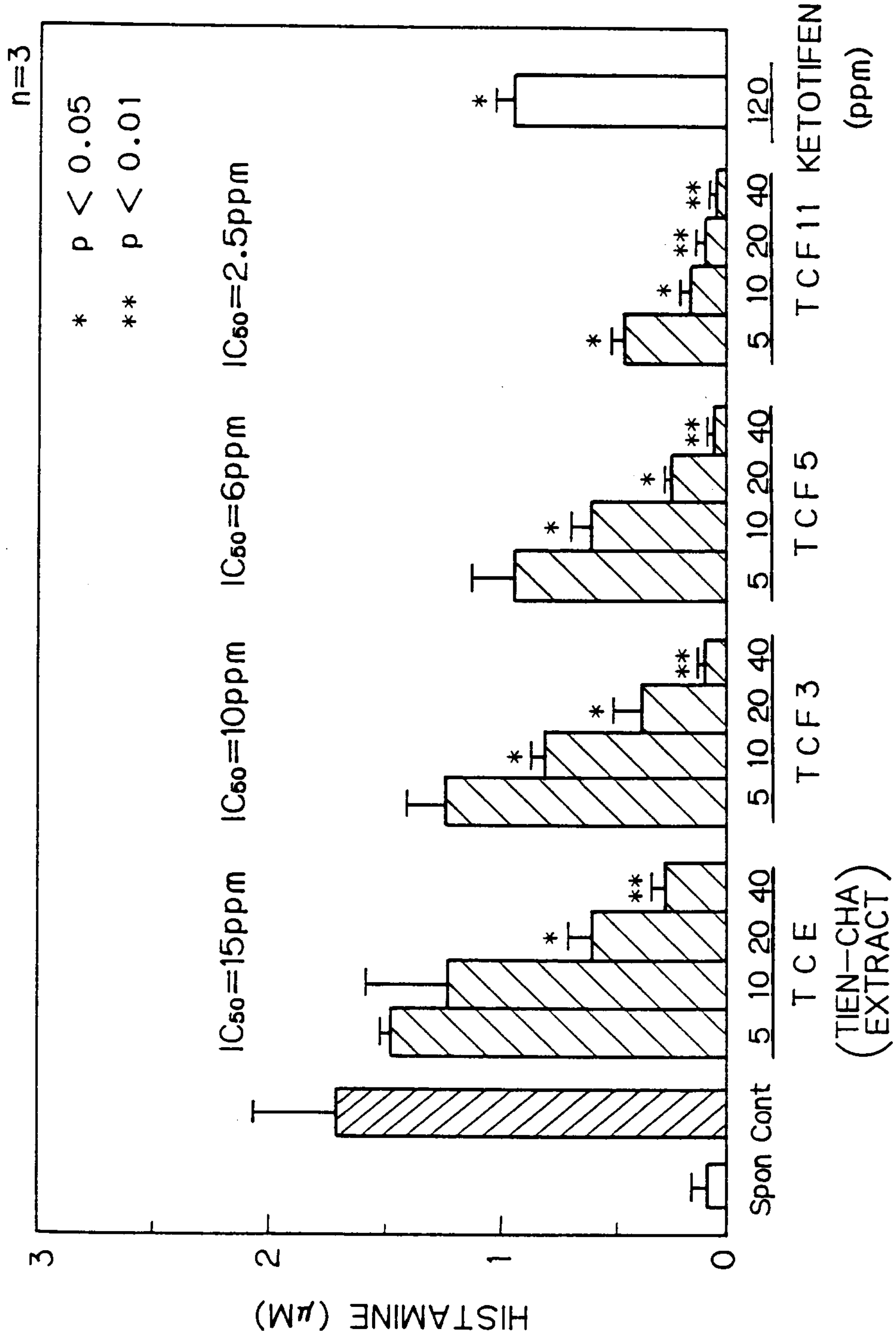

n=3
* p < 0.05
** p < 0.01
IC50=15ppm
IC50=10ppm
IC50=6ppm
IC50=2.5ppm
HISTAMINE (µM)
0
1
2
3
Spon
Cont
5 10 20 40
T C E
(TIEN-CHA EXTRACT)
5 10 20 40
T C F 3
5 10 20 40
T C F 5
5 10 20 40
T C F 11
120
KETOTIFEN
(ppm)

Fig. 5

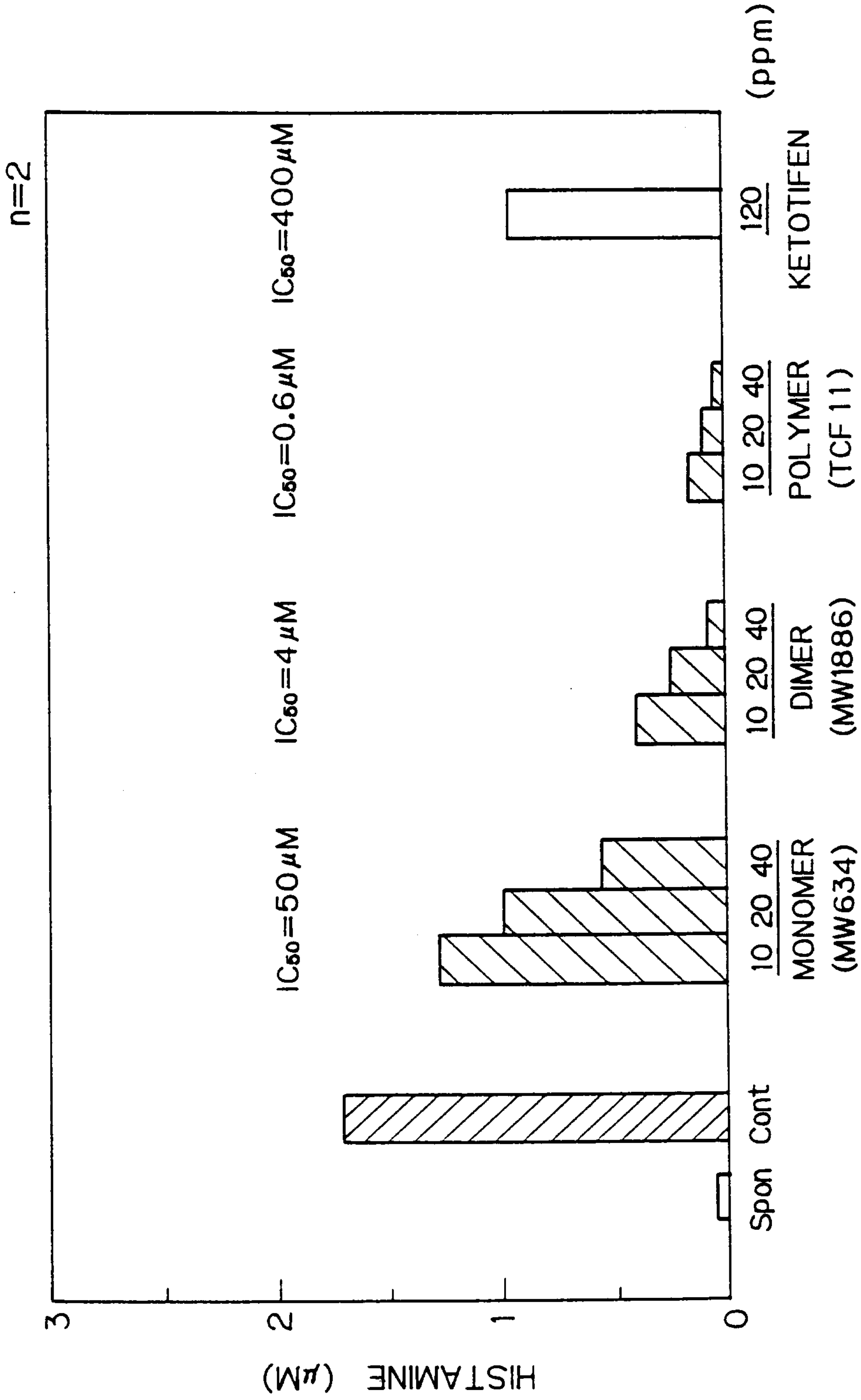

n=2
IC50=50μM
IC50=4μM
IC50=0.6μM
IC50=400μM
HISTAMINE (μM)
0
1
2
3
Spon
Cont
10 20 40
MONOMER
(MW634)
10 20 40
DIMER
(MW1886)
10 20 40
POLYMER
(TCF 11)
120
KETOTIFEN
(ppm)

Fig. 6

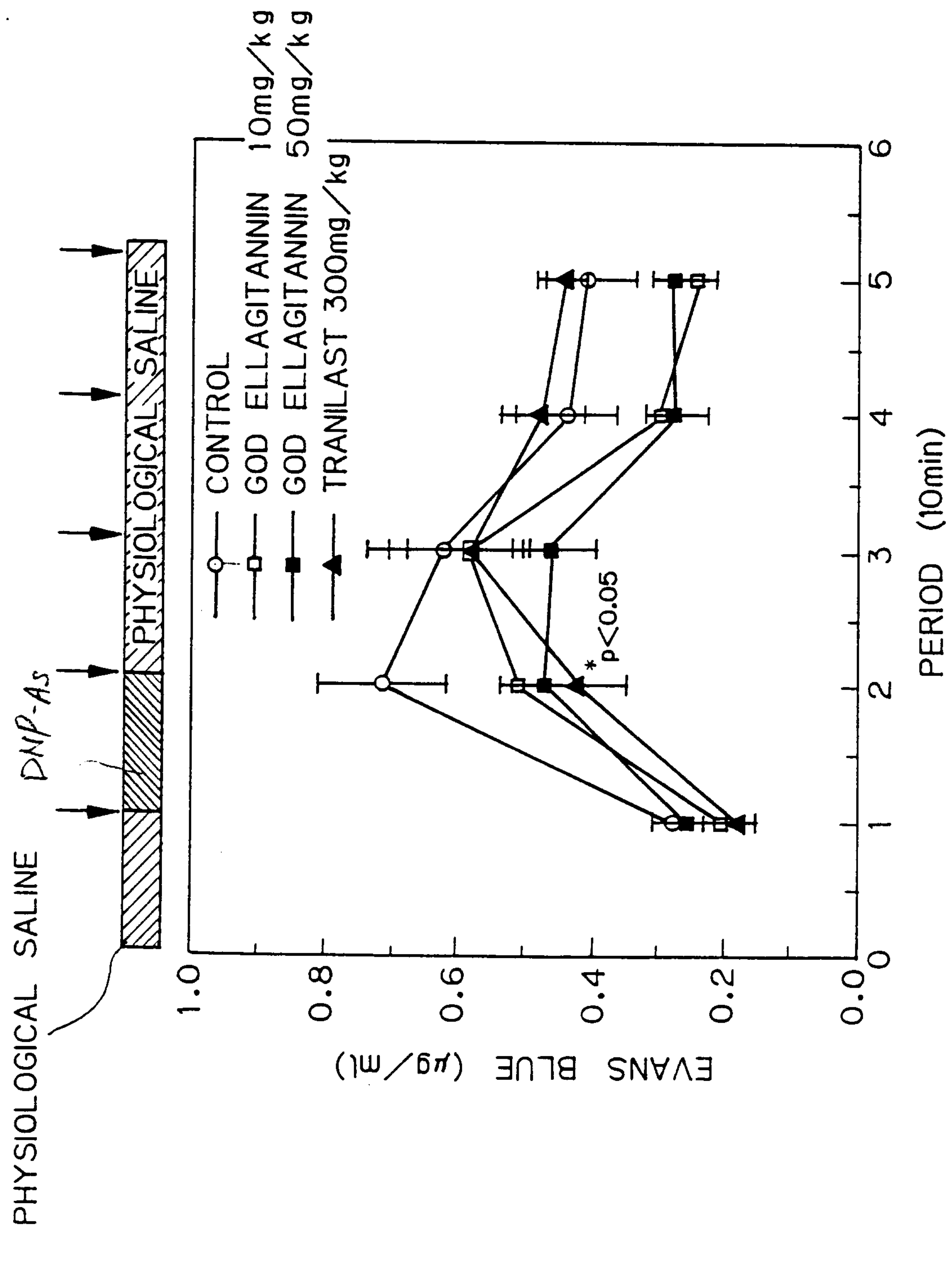
PHYSIOLOGICAL SALINE
DNP-As
PHYSIOLOGICAL SALINE
CONTROL
GOD ELLAGITANNIN 10mg/kg
GOD ELLAGITANNIN 50mg/kg
TRANILAST 300mg/kg
*p<0.05
EVANS BLUE (μg/ml)
PERIOD (10min)

*Fig. 7*

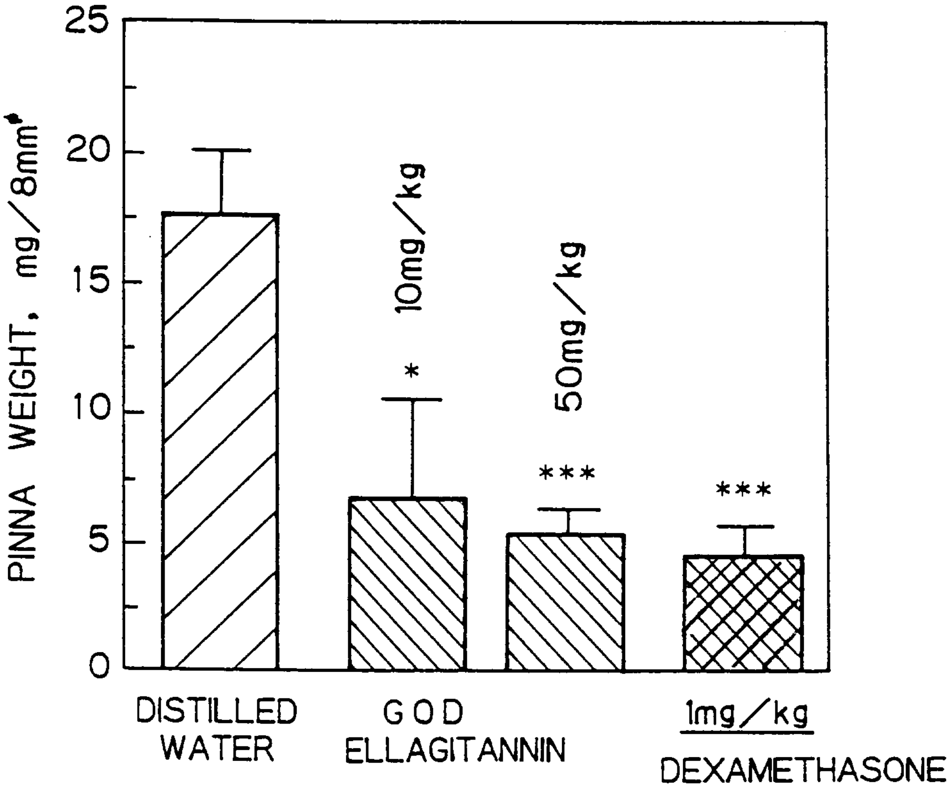


mean±SE (n=10)
*p< 0.05, *** ; p < 0.001

Fig. 8

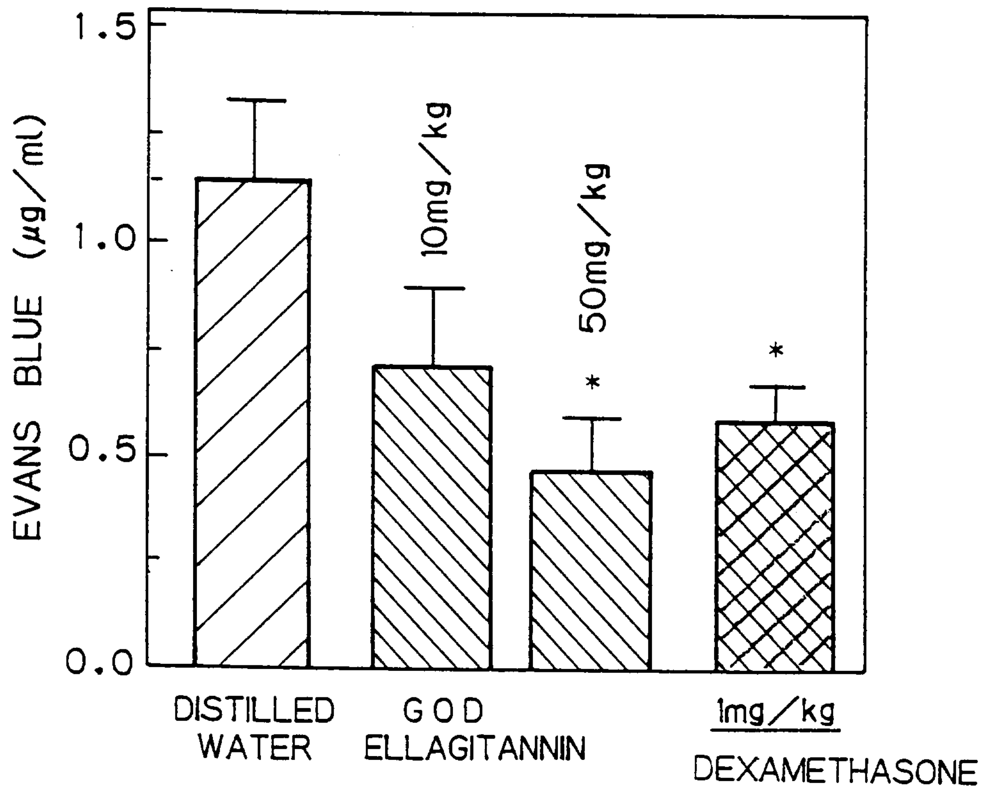


mean±SE (n=10)

*$p < 0.05$

*Fig. 9*

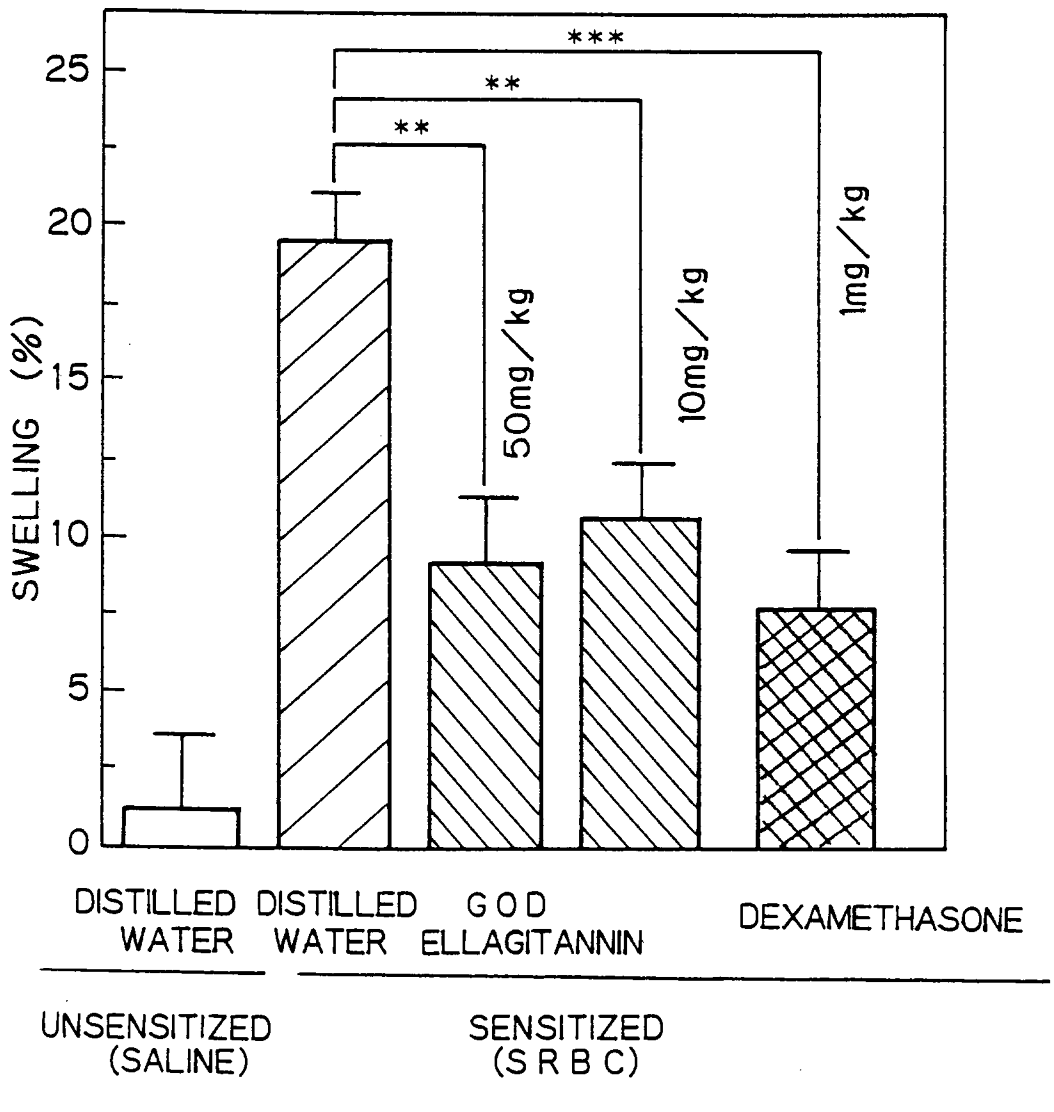


mean±SE (n=8~10)

** $p < 0.01$